Europäisches Patentamt

European Patent Office

Office européen des brevets

(19)

(11) Veröffentlichungsnummer: **0 150 424 B1**

# EUROPÄISCHE PATENTSCHRIFT

(12)

(45) Veröffentlichungstag der Patentschrift: **28.08.91**

(21) Anmeldenummer: **84115655.7**

(22) Anmeldetag: **17.12.84**

(51) Int. Cl.⁵: **C12N 15/00**, C12N 9/70,
C12N 1/20, A61K 37/54,
//(C12N1/20,C12R1:46)

(54) **Unikale, monogenetische Streptokinase, ihre Herstellung und Verwendung.**

(30) Priorität: **16.12.83 DD 258031**

(43) Veröffentlichungstag der Anmeldung:
**07.08.85 Patentblatt 85/32**

(45) Bekanntmachung des Hinweises auf die
Patenterteilung:
**28.08.91 Patentblatt 91/35**

(84) Benannte Vertragsstaaten:
**AT BE CH DE FR GB IT LI NL SE**

(56) Entgegenhaltungen:
**EP-A- 0 151 337**
**GB-A- 967 628**
**GB-A- 2 091 268**

**CHEMICAL ABSTRACTS, Band 94, Nr. 19, 11.
May 1981, Seite 351, Nr. 153326b, Columbus,
Ohio, US; H. MALKE et al.: "Molecular cloning in streptococci: physical mapping of
the vehicle plasmid pSM10 and demonstration of intergroup DNA transfer" & MGG,
MOL. GEN. GENET. 1981, 181(2), 259-67**

(73) Patentinhaber: **THE BOARD OF REGENTS OF
THE UNIVERSITY OF OKLAHOMA
1000 Asp Avenue
Norman, Oklahoma(US)**

(72) Erfinder: **Malke, Horst, Dr. sc. nat.
Edwin-Morgner-Strasse 20
O-6900 Jena-Lobeda(DE)**
Erfinder: **Gerlach, Dieter, Dr. rer. nat.
Otto-Grotewohl-Strasse 35
O-5320 Apolda(DE)**
Erfinder: **Köhler, Werner, Prof. Dr. Dr. rer. nat.
Reichweinstrasse 26
O-6900 Jena(DE)**
Erfinder: **Rattke, Wilfried, Dr. rer. nat.
Karl-Marx-Strasse 7
O-8122 Radebeul(DE)**
Erfinder: **Volzke, Klaus, Dr. rer. nat.
Gubenerstrasse 47
O-8036 Dresden(DE)**

BIOCHEMISTRY, Band 21, 1982, Seiten 6620-6625, American Chemical Society; K.W. JACKSON et al.: "Complete amino acid sequence of streptokinase and its homology with serine proteases"

PROC. NATL. ACAD. SCI. USA, Band 81, Juni 1984, Seiten 3557-3561; H. MALKE et al.: "Streptokinase: cloning, expression, and excretion by Escherichia coli"

MOL. GEN. GENET, Band 196, 1984, Seiten 360-363, Springer-Verlag; H. MALKE et al.: "Expression of a streptokinase gene from streptococcus equisimilis in streptococcus sanguis"

BIOCHEMISTRY, Band 13, Nr. 10, 1974, Seiten 2063-2070; W.J. BROCKWAY et al.: "A characterization of native streptokinase and altered streptokinase isolated from a human plasminogen activator complex"

THE JOURNAL OF BIOLOGICAL CHEMISTRY, Band 251, Nr. 13, 10. Juli 1976, Seiten 3913-3920, US; G.E. SIEFRING et al.: "Interaction of streptokinase with plasminogen. Isolation and characterization of a streptokinase degradation product"

BIOCHEMISTRY, Band 25, Nr. 1, 1986, Seiten 108-114, Washington, DC, US; K.W. JACKSON et al.: "Active streptokinase from the cloned gene in streptococcus sanguis Is without the carboxyl-terminal 32 residues"

(74) Vertreter: **Dost, Wolfgang, Dr.rer.nat.,Dipl.-Chem. et al Patent- & Rechtsanwälte Bardehle . Pagenberg . Dost . Altenburg . Frohwitter . Geissler & Partner Galileiplatz 1 Postfach 86 06 20 W-8000 München 86(DE)**

## Beschreibung

Die Erfindung betrifft eine unikale Streptokinase, ein Verfahren zu ihrer Herstellung durch Submersfermentation, einen zu ihrer Herstellung geeigneten Streptococcus-Stamm sowie ihre pharmakologische Verwendung. Das Anwendungsgebiet der Erfindung liegt entsprechend in der mikrobiologisch-pharmazeutischen Industrie.

Streptokinase ist ein in der Humanmedizin eingeführtes Therapeutikum. Dieses Protein katalysiert die Umwandlung des enzymatisch inerten Plasminogens des Blutplasmas in enzymatisch aktives Plasmin, das durch begrenzte Proteolyse das Fibrinnetzwerk von Blutgerinnseln solubilisiert. Auf dieser fibrinolytischen Reaktion basiert der Einsatz von Streptokinase in der klinischen Medizin als bedeutsames Therapeutikum zur Behandlung thromboembolischer Gefäßverschlüsse (venöse und arterielle Thrombosen, Thrombophlebitiden, Lungenembolien, Gehirnembolien etc.).

Für die Streptokinase-Herstellung sind bisher Submersfermentationsverfahren bekannt, bei denen Stämme aus der Gruppe der pathogenen Streptokokken der serologischen Gruppen A, C und G, vorzugsweise jedoch Stämme des Genus Streptococcus aus der serologischen Gruppe C, so beispielsweise Stämme der Species Streptococcus equisimilis, als Produzenten eingesetzt werden. Auf diese Weise hergestellte Streptokinasen werden beispielsweise unter den Handelsnamen Awelysin[R] (vgl. DD-A-11 096), Streptase[R] oder Kabikinase[R] vertrieben.

Für diese Fermentationsverfahren zur Herstellung von Streptokinase sind folgende Nachteile kennzeichnend:

- Die bisher eingesetzten Streptokinasebildner liefern hohe Mengen bakterieller Toxine, z.B. Streptolysin C und Streptolysin S;
- bei den bisherigen mikrobiellen Herstellungsverfahren kann der Wirkstoff Streptokinase nur nach aufwendigen Reinigungsschritten in pyrogenfreier Form erhalten werden;
- bei den gegenwärtig eingesetzten Streptokinasebildnern mußten selbst mit konventionellen genetischen Methoden durchgeführte Maßnahmen zur Steigerung der Syntheseleistung bisher unterbleiben, da auf keine Kenntnisse über die Genetik der Bildner im allgemeinen und über die genetischen Grundlagen der Streptokinase-Synthese im besonderen zurückgegriffen werden konnte.

Der Erfindung liegt die Aufgabe zugrunde, eine neue, mit Sicherheit unikale Streptokinase, ein technologisch einfaches Fermentationsverfahren zu ihrer Herstellung, einen dafür einsetzbaren neuen Mikroorganismus sowie ihre pharmazeutische Verwendung anzugeben. Das Verfahren soll dabei die Herstellung von Streptokinase mit Hilfe eines heterologen, bisher als Streptokinase-Bildner nicht bekannten Mikroorganismus aus leicht zugänglichen und billigen Einsatzstoffen erlauben.

Die Aufgabe wird anspruchsgemäß gelöst. Vorteilhafte Weiterbildungen sind Gegenstand der abhangigen Ansprüche.

Der erfindungsgemäße Mikroorganismus ist der Stamm Streptococcus sanguis (Challis) (pSM 752), der hinterlegt ist bei der Deutschen Sammlung von Mikroorganismen, DSM, Göttingen, jetzt D-3300 Braunschweig, DE, Mascheroder Weg 1b, unter der Hinterlegungs bezeichnung DSM 3140. Dieser Streptokinase produzierende Stamm besitzt ein gentechnologisch eingeführtes Streptokinasegen aus dem Donorstamm Streptococcus equisimilis H46A und ist durch Transformation mit dem Plasmid pSM 752 erhalten.

Die erfindungsgemäße Streptokinase besitzt einen isoelektrischen Punkt im Bereich von 5,0 bis 5,5 sowie eine Molekülmasse von etwa 42.000. Das herkömmliche Streptokinaseprodukt Awelysin[R] weist eine Molekülmasse von ca. 47.000 auf.

Beim Verfahren gemäß der Erfindung zur Herstellung dieser Streptokinase wird bei der Fermentation ein heterologer Streptokokkenstamm der serologischen Gruppe H und vorzugsweise ein Stamm der Art Streptococcus sanguis und besonders bevorzugt ein mit dem Plasmid pSM 752 transformierter Stamm von Streptococcus sanguis und insbesondere der oben angegebene Stamm Streptococcus sanguis (Challis) (pSM 752) eingesetzt, der bei DSM unter der Hinterlegungs bezeichnung DSM 3140 hinterlegt ist.

Bei diesem Verfahren wird der betreffende Streptokokkenstamm unter sterilen Kulturbedingungen in einem flüssigen Nährmedium mit Kohlenstoff- und Stickstoffquellen sowie Mineralsalzen gezüchtet. Der eingesetzte Streptokokkenstamm wird bei einer Temperatur von 25 bis 40 °C, vorzugsweise bei 30 °C, und bei einem pH-Wert im Bereich von pH 6,0 bis pH 8,0, vorzugsweise bei pH 7,2, für die Dauer von 10 bis 36 h und vorzugsweise 24 h fermentiert. Das einzusetzende Nährmedium enthält als Kohlenstoffquelle Zucker, vorzugsweise Glucose, als Stickstoffquelle hydrolysiertes Casein, vorzugsweise jedoch ein Hefeautolysat, sowie übliche Mineralsalze und gegebenenfalls Vitaminzusätze.

Der Zuckergehalt ist während der Fermentation konstant auf einen Wert von 0,2 bis 2 % und vorzugsweise auf 1 % einzustellen. Das als Stickstoffquelle bevorzugt eingesetzte Hefeautolysat kann einen Stickstoffgehalt von 0,1 bis 0,8 % und bevorzugt 0,2 bis 0,4 % aufweisen. Die Fermentation kann in Rundkolben verschiedenen Inhalts, in Glasfermentern sowie in V2A-Stahltanks erfolgen.

Die Isolierung der erfindungsgemäß nach dieser Verfahrensweise erhältlichen neuen unikalen Streptokinase aus der Fermentationslösung erfolgt auf an sich bekannte Weise, beispielsweise nach dem in der Patentschrift DD-A-121 522 beschriebenen Verfahren.

Die gewonnene neue Streptokinase aktiviert Human-Plasminogen zu Plasmin ; sie präzipitiert mit spezifischem Antistreptokinase-Serum vom Kaninchen.

Die Erfindung weist folgende Vorteile auf:
- Erhalten wird ein unikales, durch **ein** Gen determiniertes Streptokinase-Produkt.
- Streptokokken-Toxine, wie beispielsweise Hämolysine, werden im vorliegenden Verfahren **nicht** mit gebildet.

Das folgende Ausführungsbeispiel erläutert das erfindungsgemäße Verfahren.

Beispiel

Ein Streptokokkenstamm (DSM 3140) wird auf 2 % Agar mit 3,7 g/100 ml Brain heart infusion plus 5 $\mu$g/ml Erythromycin gehalten. Hiervon werden 5 ml Hefeautolysatnährboden als 1. Vorkultur beimpft und nach 20 h Inkubation bei 37 °C zur Beimpfung der 2. Vorkultur von 250 ml Hefeautolysatnährboden verwendet. Beide Vorkulturen enthalten 5 $\mu$g/ml Erythromycin.

Die Kulturlösung besteht aus 40 g/l Hefeautolysat in Wasser entsprechend DD-A-111 096. Sie wird jeweils durch Autoklavieren sterilisiert; anschließend wird 1 % Glucose in Form einer sterilen 50-%igen Lösung zugeführt.

Mit 250 ml Vorkultur werden 50 l Hauptkultur beimpft. Gezüchtet wird unter Rühren bei 30 °C ohne Antibiotikazusatz. Der pH-Wert von 7,2 und der Glucosespiegel von 1 % werden automatisch durch Zupumpen von 20 g/100 ml Natronlauge bzw. 50 g/100 ml Glucoselösung geregelt bzw. aufrechterhalten. Die nach 24 h erhaltene Kulturlösung enthält 1600 IE/ml Streptokinase; ihre Aufarbeitung wird in Anlehnung an DD-A-121 522 durchgeführt, wobei der Konzentrierungsschritt ähnlich dem Verfahren von DD-A-126 342 mit Kieselgel erfolgt. Im einzelnen wird wie folgt verfahren:

50 l Streptokokkenkultur werden zur Abtötung der Kokken mit 250 ml 30-%igem $H_2O_2$ versetzt und 1 h gerührt. Anschließend werden die Kokken abgetrennt. Aus dem Kulturfiltrat wird die Streptokinase durch Einrühren von 1 kg Kieselgel B I (0,1 bis 0,4 mm) adsorbiert. Vom intensiv mit Wasser gewaschenen Kieselgel wird die Streptokinase mit 5 l einer 1-%igen Lösung von $Na_2CO_3$ in 10 % Ethanol-Wasser eluiert. Aus dem Eluat wird die Streptokinase durch Zugabe von 500 g NaCl und pH-Erniedrigung auf 4,0 gefällt. Der abgetrennte Niederschlag wird in Wasser bei pH 8,0 gelöst (ca. 300 ml). Die Lösung wird dreimal mit frisch gefälltem $CaHPO_4$-Gel bei pH = 7,0 gerührt (ca. 4 g). Die Streptokinase wird hieraus mit Ammoniumsulfat zwischen 25 % und 45 % Sättigung gefällt. Der abgetrennte und in $H_2O$ gelöste Ammoniumsulfatniederschlag wird schließlich an Sephacryl-S200 (Säule 50 cm x 5 cm; 0,5 M NaCl; 0,01 M Phosphatpuffer pH 7,0) fraktioniert, worauf die streptokinasehaltigen Fraktionen vereinigt werden.

Ausbeute: 170 ml mit einer Streptokinaseaktivität von 9,0 $\cdot$ $10^6$ IE und ca. 50.000 IE/mg Protein.

Die Charakterisierung erfolgte durch Natriumdodecylsulfat-Elektrophorese nach WEBER, K., und OSBURN, M., J. Biol. Chem. 244 (1969) 4406; hierbei wurde eine einheitliche Substanz mit einer Molekülmasse von etwa 42.000 gefunden.

**Patentansprüche**

1. Streptokinase erzeugender Streptococcus sanguis (Challis) (pSM 752) mit gentechnologisch eingeführtem Streptokinase-Gen aus dem Donorstamm Streptococcus equisimilis H46A, hinterlegt bei der Hinterlegungsstelle DSM, Göttingen, jetzt Braunschweig, DE, unter der Hinterlegungsbezeichnung DSM 3140.

2. Verfahren zur fermentativen Herstellung einer unikalen Streptokinase, dadurch gekennzeichnet, daß zur Fermentation ein mit dem gentechnologisch gewonnen, aus dem hinterlegten Stamm DSM 3140 erhältlichen Plasmid pSM 752 transformierter Streptokokken-Stamm der serologischen Gruppe H eingesetzt wird.

3. Verfahren nach Anspruch 2, dadurch gekennzeichnet, daß zur Fermentation ein mit dem Plasmid pSM 752 transformierter Stamm der Art Streptococcus sanguis eingesetzt wird.

4. Verfahren nach Anspruch 2 oder 3, dadurch gekennzeichnet, daß zur Fermentation der Stamm Streptococcus sanguis (Challis) (pSM 752) eingesetzt wird, der hinterlegt ist bei der Hinterlegungsstelle DSM, Göttingen, jetzt Braunschweig, DE, unter der Hinterlegungsbezeichnung DSM 3140.

5. Streptokinase, gekennzeichnet durch eine Molekülmasse von etwa 42 000 sowie einen isoelektrischen Punkt im Bereich von 5,0 bis 5,5 und erhältlich nach dem Verfahren nach Anspruch 4.

6. Pharmazeutische Mittel, gekennzeichnet durch eine Streptokinase nach Anspruch 5 als Wirkstoff.

**Claims**

1. Streptokinase-producing Streptococcus sanguis (Challis) (pSM752) having a genetechnologically introduced streptokinase gene from the donor strain Streptococcus equisimilis H46A, deposited with the depositary DSM, Göttingen, now Braunschweig, DE, under the depositary designation DSM 3140.

2. A process for fermantative production of a Unique streptokinase, characterized by using for fermentation a streptococcus strain of the serological group H transformed with the genetechnologically obtained plasmid pSM 752 obtainable from the deposited strain DSM 3140.

3. The process of claim 2, characterized by utilizing for fermentation a stain of the species Streptococcus sanguis transformed with plasmid pSM 752.

4. The process of claim 2 or 3, characterized by utilizing for fermentation the strain Streptococcus sanguis (Challis) (pSM 752), deposited with the depositary DSM Göttingen, now Braunschweig, DE, under the depositary designation DSM 3140.

5. Streptokinase, characterized by a molecular mass of about 42.000 and an isoelectrical point in the range from 5.0 to 5.5, and obtainable according to the process of claim 4.

6. Pharmaceutical compositions, characterized by a streptokinase according to claim 5 as active ingredient.

**Revendications**

1. Streptococcus sanguis (Challis) (pSM 752) produisant de la streptokinase, comportant le gène de la streptokinase inséré par génie génétique à partir de la souche donneuse Streptococcus equisimilis H46A, déposée au centre de dépôt DSM, Göttingen, maintenant Braunschweig, DE, sous le numéro de dépot DSM 3140.

2. Procédé de préparation par fermentation d'une streptokinase unique, caractérisé en ce que l'on utilise pour la fermentation une souche de streptocoques appartenant au groupe sérologique H transformée par le plasmide pSM 752, obtenu par génie génétique, disponible à partir de la souche déposée DSM 3140.

3. Procédé selon la revendication 2, caractérisé en ce que l'on utilise pour la fermentation une souche du genre Streptococcus sanguis tranformée avec le plasmide PSM 752.

4. Procédé selon la revendication 2 ou 3, caractérisé en ce que l'on utilise pour la fermentation la souche Streptococcus sanguis (Challis) (pSM 752), qui est déposée au centre de dépôt DSM, Göttingen, maintenant Braunschweig, DE, sous le numéro de dépôt DSM 3140.

5. Streptokinase caractérisée par un poids moléculaire d'environ 42 000 ainsi qu'un point isoélectrique dans la gamme de 5,0 à 5,5 et pouvant être obtenue par le procédé selon la revendication 4.

6. Produit pharmaceutique, caractérisé en ce qu'il comporte comme ingrédient actif une streptokinase selon la revendication 5.